# EUROPEAN PATENT SPECIFICATION

(11) **EP 3 303 608 B1**
(45) Date of publication and mention of the grant of the patent: **01.07.2020**
(21) Application number: 16799394.8
(22) Date of filing: 24.05.2016
(51) Int. Cl.: C12Q 1/34, B01L 3/00, C12Q 1/00

(54) **ENZYME ACTIVITY ASSAY SYSTEM AND DEVICES**
ENZYMAKTIVITÄTTESTSYSTEM UND -VORRICHTUNGEN
SYSTÈME ET DISPOSITIFS DE DOSAGE D'ACTIVITÉ ENZYMATIQUE

(30) Priority: 26.05.2015 DK 201570311
(43) Date of publication of application: 11.04.2018
(73) Proprietor: Københavns Universitet (KU), 1165 København K (DK); Danmarks Tekniske Universitet, 2800 Lyngby (DK)
(72) Inventor: KRACUN, Stjepan Kresimir, 2500 Valby (DK); SCHÜCKEL, Julia, 1871 Frederiksberg C (DK); WILLATS, William George Tycho, Newcastle upon Tyne NE15 0NS (DK); CLAUSEN, Mads Hartvig, 2400 Copenhagen NV (DK)
(74) Representative: Plougmann Vingtoft a/s
(86) International application number: PCT/DK2016/050144
(87) International publication number: WO 2016/188533

(56) References cited:
- WO-A1-2014/121243
- WO-A1-2015/036000
- WO-A2-2004/076054
- CN-A- 104 062 271
- GB-A- 2 085 159
- JP-A- 2009 258 136
- KRA?UN S.K ET AL.: 'A new generation of versatile chromogenic substrates for high-throughput analysis of biomass-degrading enzymes' BIOTECHNOLOGY FOR BIOFUELS vol. 8, no. 70, 2015, pages 1 - 16, XP021221802
- KIYONAKA S. ET AL.: 'Semi-wet peptide/protein array using supramolecular hydrogel' NATURE MATERIALS vol. 3, no. 1, 2004, pages 58 - 64, XP002504612

## Description

The work leading to this invention has received funding from the European Union Seventh Framework Program (FP7/2007-2013) under grant agreement n°263916.

### TECHNICAL FIELD

The invention relates to an enzyme activity assay system and devices for determining biopolymer enzyme degrading activity in a liquid sample. The invention also relates to devices for performing biopolymer enzyme degrading activity assays.

### BACKGROUND ART

Enzymes constitute a family of proteins involved in catalyzing chemical reactions within living organisms. As a result of their importance, there are numerous situations in which it is necessary and/or beneficial to measure enzyme levels, and importantly, enzyme activity.

Within the enzyme family, there are many classes of enzyme that act by facilitating substrate cleavage, for example through hydrolysis or elimination. Such substrate cleavage is usually referred to as substrate degradation.

Enzymes that degrade or modify polysaccharides are widespread in pro- and eukaryotes and have multiple biological roles and biotechnological applications. Recent advances in genome and secretome sequencing, together with associated bioinformatic tools have enabled large numbers of carbohydrate acting enzymes to be putatively identified. However, there is a paucity of methods for rapidly determination of the biochemical activities of these enzymes.

In many processes e.g. industrial processes it is desired to control degradation of polysaccharide, such as in biofuel production or other processes involving fermenting sugars and carbohydrates from plant material by using enzymes with selected properties.

Enzyme assays comprising testing a sample's capability of breaking down a cross-linked polysaccharide substrate have been known for more than 30 years.

For example US 4,066,509 describes a method for determining the activity, or concentration, of a hydrolyzing enzyme, *e.g.,* an *endo*-enzyme*.* The activity is determined by providing a detection layer comprising a carrier matrix and a water-insoluble substrate such as starch, dextran, cellulose and albumin which is marked with a detectable marker, *e.g*., dyed and cross-linked to form a gel, and which substrate, upon action of a hydrolyzing enzyme thereon, is broken down into soluble fragments capable of diffusing through the carrier matrix to cause formation of an unmarked area in said detection layer; and measuring the size of the unmarked area as a measure of the activity of concentration of said enzyme.

US 5,496,707 describes an assay method for hemicellulases comprising a) directly dyeing, using a reactive dye, an insoluble natural product, or a modified form of a natural fibre material; and b) adding the enzyme to the dyed product produced in step a) and, after a specific incubation period, separating the liquid component from the insoluble dyed product, e.g. by a simple filtration, and determining the amount of dyestuff liberated in the separated solution by spectrophotometric means.

US2010028916 discloses a method for the detection of an enzyme E1 in a liquid sample comprising the steps of: a) providing a complex (Sa-Sb-M), wherein (Sa-Sb) is a substrate S of E1 cleavable into Sa and Sb by E1, and M is a marker linked to Sb, b) incubating the sample with the complex under conditions enabling the cleavage of S into Sa and Sb by E1, c) separating non-cleaved complex (Sa-Sb-M) from the sample, and d) measuring M in the sample.

WO 15036000 discloses an enzyme substrate comprising an enzymatically degradable microparticulate plant material having a polysaccharide content in the range of 35 percent (w/w) to 95 percent (w/w) and comprising a marker which upon degradation is released. In an example the chromogenic cross-linked polysaccharide substrates is used in an enzyme assay performed in a 96 well plate.

A method for qualitatively determination of polysaccharide degrading enzymes was described by Ten et al. "Novel insoluble dye-labeled substrates for screening insulin-degrading microorganism". Journal of Microbiological Methods 69 (2007) 353-357. The assay was performed by providing a gelled and dyed insulin substrate, which was inoculated in agar plate. Colonies of insulin degrading microorganism was detected as haloes in the agar plate.

A chromogenic hydrogel substrate and an assay for detecting enzyme activity of a sample are described in "A new generation of versatile chromogenic multi-colored substrates for high-throughput analysis of biomass-degrading enzymes" by Kračun et al. Biotechnology for biofuels (2015) Vol. 8, No70, pages 1-16. The chromogenic hydrogel substrate is obtained from polysaccharides which are dyed and crosslinked. The substrate was dispensed by syringes into 96-well filter plates. A solution comprising enzyme or buffer was added to each well. After an incubation time it was observed if soluble dyed oligosaccharide products would be released.

CN 104 062 271 discloses a method of detecting a substance in water. The method comprises providing a detection device comprising a dry gel of polymers. The gel is called a xerogel, but no other characteristics are given. The gel is loaded with non-water-soluble fluorescent. The gel is encapsulated in a polymer material including a substrate with a permeable polymer film cover, where at least one of these is transparent. In use, a first emission spectrum measurement of the fluorescent probes at the dry gel surface is obtained. The gel is immersed in an aqueous solution comprising the substance to be detected for a selected time where after the water is removed. Thereafter a second emission spectrum measurement of the fluorescent probes at the gel surface is obtained. By comparing the first and the second emission spectrums, the amount of the substance in the aqueous solution can be determined.

GB 2 085 159 discloses a multilayer chemical analysis element comprising a support layer forming a light-transmissible, water-impermeable support; a first reagent layer on the support comprising a color-forming reaction layer comprised of a chromogen compound capable of forming a dye upon reaction with a dye-forming reactive group; and a second reagent layer comprising a substrate layer comprised of a non-diffusible substrate comprising the dye-forming reactive group, which is substantially colorless and is capable of forming a dye upon reaction with the chromogen compound, the substrates are capable of reacting with an analyte to form a substantially colorless diffusible compound which contains the dye-forming reactive group.

### DISCLOSURE OF INVENTION

An object of the present invention is to provide an enzyme activity assay device suitable for determination of biopolymer enzyme degrading activity in a liquid sample, which assay system and device is both fast and simple to use.

In an aspect of the disclosure it is an object to provide an enzyme activity assay system suitable for determination of biopolymer enzyme degrading activity in a liquid sample, which assay system, is both fast and simple to use and which further can be used to perform quantitative determinations i.e. a degree of enzyme activity relative to a selected reference.

In an aspect of the invention it is an object to provide an enzyme activity assay device suitable for determination of biopolymer enzyme degrading activity in a liquid sample, which assay system is both fast and simple to use and which does not require any expensive read-out equipment.

These and other objects have been solved by the inventions or embodiments thereof as defined in the claims and as described herein below.

It has been found that the inventions or embodiments thereof have a number of additional advantages which will be clear to the skilled person from the following description.

The enzyme activity device of the invention is suitably for determination of biopolymer enzyme degrading activity in a liquid sample. The enzyme activity device comprises a biopolymer substrate and a solid support structure supporting said biopolymer substrate. The biopolymer substrate comprises a dyed and water insoluble xerogel comprising a network of cross-linked biopolymers.

The term "Biopolymers" as used herein is meant to designate polymers produced by or producible by living organisms as well as derivatives, fractions, combinations or combinations thereof including modified polymers. The biopolymers contain monomeric units that are covalently bound (polymerized) to form larger structures; in other words, they are polymeric biomolecules, preferably in form of polynucleotides (such as deoxyribonucleic acid (DNA) and ribonucleic acid (RNA), polypeptides and proteins (such as casein, bovine serum albumin, collagen and others), polysaccharides (cell wall polysaccharides from plants and fungi or extracellular matrix (ECM) polysaccharides such as glycosaminoglycans and others) or any combinations or modifications thereof. As mentioned the biopolymers may be modified e.g. by attaching moieties or similar which does not destroy the biopolymers degradability for natural enzymes.

The substrate may comprise any combination of biopolymers and will usually be constructed to have a suitable degradability for the enzyme that is to be tested for.

The biopolymers are cross-linked, where the degree of cross-linking advantageously is selected such that the xerogel is water insoluble in non-degraded state. At the same time it is desirable that the cross-linking degree is not too high because a too high crosslinking may prevent the enzyme from having sufficient access to degrading the biopolymers. For a given biopolymer or biopolymer combination the skilled person will be able to find a suitable cross-linking degree.

The term "water insoluble" is herein used to mean that the substrate should not be dissolved in demineralized water at 25 °C. Advantageously the biopolymer substrate is sufficiently stable and water insoluble to maintain its cross-linked structure when immersed in demineralized water at 25 °C and optionally positioned on a lab vibrating table for at least about 30 minutes.

The term "substantially" should herein be taken to mean that ordinary product variances and tolerances are comprised.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Throughout the description or claims, the singular encompasses the plural unless otherwise specified or required by the context.

According to an embodiment of the invention is has been found that by use of the enzyme activity device according to the claims comprising the substrate comprising or consisting of a xerogel a very accurate and reliable enzyme assay may be performed which is even more accurate in use than using the prior art substrates. It has been found that by providing the biopolymers in form of a cross-linked construction in a gelled form which is dried to form a xerogel, the amount of liquid within the xerogel when re-moistured is highly reduced compared to the amount of liquid in the original gel. It has further been found that this reduction of liquid results in a much more accurate assay.

The solid support structure can in principle be any kind of support structure which is sufficiently stable to support the xerogel in dry or in re-moistured condition. This means accordingly that the solid support structure should be water insoluble and advantageously substantially rigid, preferably such that it is not bending when using the enzyme activity device according to its ordinary use.

Advantageously the solid support structure is selected from a well containing test plate, a tube, a lateral flow device or a microfluidic device. These different types of support structures are disclosed further below.

Preferably the solid support structure is substantially rigid such that it can be held manually or by an apparatus during use. It has been found to be advantageous to make the solid support structure fully or partly from a polymer. Preferable the solid support structure is obtainable from injection molding or similar process for mass production.

As mentioned the biopolymer substrate may comprise polymeric biomolecules of any suitable type or types. Generally it is desired that the biopolymer substrate is composed for testing of the enzyme activity of one or more target enzymes.

In an embodiment, the biopolymer substrate comprises cross-linked polymeric biomolecules selected from polynucleotides, polypeptides, polysaccharides or any combinations thereof.

The biopolymers may advantageously be obtained from natural sources e.g. in form of fractions or derivatives or modifications thereof. In some situations it is desired to artificially produce the biopolymers or alternatively the biopolymers may be partly obtained from natural sources and partly artificially synthetized.

In an embodiment, the polymeric biomolecules of the biopolymer substrate comprises polynucleotides selected from natural, modified or artificial sources of DNA or RNA, fractions or derivatives thereof.

The DNA or RNA, fractions or derivatives thereof may for example include commercially available DNA from salmon sperm or RNA from different yeast species.

In an embodiment, the polymeric biomolecules of the biopolymer substrate comprises polypeptides selected from casein, bovine serum albumin, collagen and others.

In an embodiment, the polymeric biomolecules of the biopolymer substrate comprises polysaccharides selected from cellulose, amylose, dextran, xylan, pectin, and glucans, mannans, galactans, arabinans.

Generally polysaccharides of plant, algal, animal and fungal origin is very suitable for use in the invention.

Table 1 lists examples of suitable biopolymers for the biopolymer substrate. The substrates of the invention are generally referred to as CBX (Chromogenic Biopolymer Xerogel) or CPX (Chromogenic Polysaccharide Xerogel and/or Chromogenic Polynucleotide Xerogel and/or Chromogenic Polysaccharide Xerogel).The term "chromogenic" is herein used to mean that reaction including enzymatic activity can be detected by extracting dye or biopolymer fragments bonded to dye.

The biopolymer substrate may be constructed to mainly be degradable by one or more selected type of enzyme(s), by ensuring the enzymatic degradable bonds are selected accordingly.

In an embodiment, the biopolymers comprises at least about 50 % of substantially identical monomeric units, such as at least about 60 %, such as at least about 70 %, such as at least about 80 %, such as at least about 90 % of substantially identical monomeric units. By having a substrate with a large amount of identical monomeric units, the biopolymer substrate will be very sensitive for enzymes with degradation activity for bonds between such monomeric units. In some situations it is desired that the biopolymer substrate is less sensitive for selected enzymes and in such cases the amount of available degradable bonds for such enzymes can be reduce by a proper selection of biopolymers and monomeric units of the biopolymers.

In an embodiment, the biopolymers comprises a plurality of enzyme degradable bonds and preferably at least about 50 % of the enzyme degradable bonds are degradable by identical enzymes, such as at least about 60 %, such as at least about 70 %, such as at least about 80 %, such as at least about 90 % of substantially identical monomeric units of the enzyme degradable bonds are degradable by one type of enzymes.

A type of enzyme includes a group of enzymes that is capable of degrading one type of chemical bond of a biopolymer substrate. A type of enzyme can e.g. be polysaccharide degrading (glycosyl hydrolase, glycosyl lyase, lytic polysaccharide monooxygenase...), protein degrading (proteases) belonging to any of the different classes (serine, threonine, cysteine, aspartate, glutamic acid and metalloproteases), deoxyribonuclease (DNase) and ribonuclease (RNase) enzymes (any enzymes capable of degrading DNA and/or RNA).

Further examples of types of enzymes are listed in table 2.

In an embodiment the biopolymer substrate comprises a plurality of enzyme degradable bonds, preferably at least about 75 %, such as at least about 80 %, such as at least about 90 %, such as substantially all of the enzyme degradable bonds are exclusively enzyme degradable by one type of enzyme.

In an embodiment the biopolymers of the substrate comprises a plurality of different monomeric units.

The xerogel is can be obtained by any suitable method comprising providing a plurality of biopolymers, dying and crosslinking the biopolymers and drying the cross-linked biopolymers.

The dying is advantageously performed prior to and/or simultaneously with the crosslinking.

In order to cross-link the biopolymers the biopolymers needs to be immersed and preferably at least partly dissolved in a liquid and usually the cross-linking requires the use of a cross linker or the cross-linking can be initiated by radiation such a UV radiation.

The crosslinking of biomolecules can for example be performed as described in Stjepan Krešimir Kračun, Julia Schückel, Bjørge Westereng, Lisbeth Thygesen, Rune Monrad, Vincent G H Eijsink, William Willats, A new generation of versatile chromogenic substrates for high-throughput analysis of biomass-degrading enzymes, Biotechnology for Biofuels, 2015, 8:70. The hydrogel based substrate - i.e. where the hydrogel has not been subjected to drying is referred to as Chromogenic Biopolymer hydrogel CBH or Chromogenic polysaccharide/polynucleotide/Polysaccharide hydrogel CPH, although the dye or color is not chromogenically formed, but merely released alone or bonded to fragment(s) of the substrate upon degradation thereof. The substrates of the invention are generally referred to as CBX or CPX as explained above.

In an embodiment the cross-linking is performed using at least one cross linker selected from homo-bi-functional cross linkers and/or hetero-bi-functional cross linkers and/or activation agents.

Examples of homo-bi-functional cross linkers includes alkane diol diglycidyl ether, such as butane-diol diglycidyl ether; dihalo-alkanes, such as dibromohexane; diamino alkanes in general, such as diaminopropane; *N,N'-*methylenebisacrylamide; succinic anhydride and divinyl sulfone.

Examples of hetero-bi-functional cross linkers include epichlorohydrin, glycidyl methacrylate and/or acrylamide.

Examples of activation agents facilitating hydrogel formation includes *N,N,N'N"*-tetramethylethylenediamine; ammonium persulfate; sodium persulfate; carbodiimides such as *N*-(3-dimethylaminopropyl)-*N'-*ethylcarbodiimide hydrochloride and *N*-cyclohexyl-*N'*-(2-morpholinoethyl) carbodiimide metho-p-toluenesulfonate .

The liquid in which the biopolymers are immersed (dissolved) may in principle be any kind of liquid which does not damage the biopolymer substrate. Preferably the liquid water, alcohol (preferably ethanol) or any mixtures thereof optionally comprising suitable additives such as surfactants. Advantageously the liquid in which the biopolymers are immersed is simple to remove after the cross-linking using air drying, oven drying (e.g. at about 30 to about 90 °C) or freeze drying e.g. at about -5 to about -150 °C, such as about -50 to about -90 °C.

The density of the xerogel depends largely on the biopolymers and the degree of cross linking. It has been found to be desirably to have a density which is less than what it would have been in a corresponding aerogel.

Advantageously the xerogel has a density of at least about 0.02 g/cm³, preferably from about 0.05 to about 0.8 g/cm³, such as from about 0.1 to about 0.5 g/cm³.

In a preferred embodiment the xerogel is obtainable from a process comprising producing a dyed biopolymer hydrogel and drying the hydrogel by freeze drying.

In an embodiment the xerogel is obtainable from a process comprising producing a dyed biopolymer gel selected from an alcogel, an aerogel or a combination thereof and drying the gel, preferably by air drying or oven drying.

In an embodiment the xerogel is obtainable from a process comprising crosslinking and dying a plurality of polymeric biomolecules in an aqueous fluid to form an hydrogel, freezing the hydrogel to a temperature of less than about -25 °C and freeze-drying the hydrogel.

In an embodiment the hydrogel is placed onto the solid support structure, thereafter the hydrogel is frozen and dried on the solid support structure. In another embodiment the hydrogel is arranged onto a tray, frozen and dried and thereafter moved to the solid support structure.

It has been found that a very and surprisingly high quality of the biopolymer substrate is obtained where the biopolymer substrate is produced from a hydrogel dried by freeze drying. Without being bound by the theory it is believed that the freeze drying of the aerogel result in damaging of at least some nanopores, thereby resulting in a more uniform pore size distribution.

Advantageously the xerogel has a porosity (gas fraction) of from about 20 % to about 85 %, such as from about 25 % to about 80 %, such as from about 30 % to about 75 %.

In an embodiment the xerogel has a volume which is about 90 % or less relative to the volume of the gel from which the xerogel is obtained by drying. Advantageously the xerogel has a volume which is about 80 % or less, such as about 70 % or less relative to the volume of the gel from which the xerogel is obtained by drying.

In an embodiment the xerogel has a surface area of at least about 100 m²/g, such as from about 150 to about 900 m²/g, such as from about 200 to about 800 m²/g.

Surface area is determined using Brunauer-Emmett-Teller (BET) Surface Area Analysis.

A portion of the dye may be spatially located within the network of cross-linked biopolymers.

The dye is chemically bound to the biopolymers. In an embodiment the dye is chemically bound to the biopolymers prior to or simultaneously with the cross-linking of the biopolymers. The enzyme active device comprising a biopolymer substrate where the dye is chemically bound to the biopolymers has been found to provide very accurate quantitative or qualitative determination of enzyme activity of a liquid sample. Any risk of false positive is very low or even absent. Since the dye is chemically bound to the biopolymers any risk that mechanical damages of the network should result in release of dye resulting in false positive determinations is practically negligible. Furthermore, there is practically no risk of washing out a too large amount of dye in a prewash, since bonded dye or biopolymer fragments comprising bonded dye will generally not be washed out without degradation of the crosslinked biopolymers.

In an embodiment the dye is covalent bound to the biopolymers of the substrate. Thereby the biopolymer substrate becomes even more stable against undesired non-enzymatic release of dye due to mechanical damage.

The dye can in principle be any kind of dye which is detectable visually and/or optically e.g. using a spectrometer, such as spectrometer detecting electromagnetic waves e.g. with wavelengths in the range from about 300 nm to about 2500 nm, such as from about 400 nm to about 1500 nm, preferably comprising the visual range from about 400 nm to about 700 nm. Such dyes are well known to the skilled person.

Examples of suitable dyes which may be used alone or in any combinations includes be chlorotriazine dyes (monochloro and dichlorotriazine dyes), monofluorochlorotriazine dyes, difluorochloropyrimidine dyes, dichloroquinoxaline dyes, trichloropyrimidine dyes, vinyl sulfone and vinyl amide dyes.

Advantageously the dye is selected from dyes that absorb in the visible spectrum and whose detection is based on absorbance as well as dyes that absorb in the UV-VIS-IR range but their detection is based on emission of fluorescence. These may include azo-dyes, diazo-dyes, anthraquinone dyes and phthalocyanine dyes among others.

Dyes whose binding to the substrate is not covalent may also be used such as calcofluor white, sirofluor and Congo Red. These dyes are in particular suitable for use where at least a portion of the dye is spatially located within the network of cross-linked biopolymers. In this way the dye in trapped within the substrate and when the biopolymer substrate is subjected to degradation the dye is release in a proportion corresponding to the degree of degradation.

In an embodiment the dye is a fluorescent dye (fluorophore).

The amount of dye may in principle be very low. Advantageously the dye is present in a sufficient amount to allow detection of even a low degree of degradation of the biopolymer substrate. The skilled person will be able to determine a suitable amount of dye for a specific substrate.

Xerogels as such may be very fragile and it has been found that the xerogel may be subjected to mechanical damage during transport or other handlings of the enzyme activity device. A mechanical damage of the xerogel may in certain situation be very disadvantageous in particular where quantitative assays are to be performed. For such enzyme activity devices it is may be desired to handling the enzyme activity device very carefully to avoid or reduce mechanical damage.

In a preferred embodiment it has been found that by adding a stabilizer to the biopolymer substrate, the resistance against mechanical damage of the biopolymer substrate is increased. By selecting a stabilizer which is soluble in a liquid that does substantially not dissolve or degrade the xerogel - such as an aqueous liquid, the stabilizer may be removed by such liquid e.g. water or a liquid comprising water, such as an aqueous buffer prior to adding a sample for test to the biopolymer substrate. Thereby the stabilizer will not influence in assay.

Advantageously the biopolymer substrate comprises a stabilizer for stabilizing the xerogel, where the stabilizer comprises at least one organic polymer and is soluble in a liquid that does not dissolve or degrade the xerogel. Preferably the stabilizer is soluble in an aqueous and/or alcoholic liquid, such as water, ethanol or a combination thereof.

In an embodiment the stabilizer further comprises up to 5 % by weight of surfactant(s) and/or dye(s). It has been found to be an advantage to include at least one dye in the stabilizer. Thereby the user can ensure that the stabilizer is fully removed prior to adding the sample.

It should be understood that the stabilizer need not be removed prior to use, in particular for assays which are merely or mainly qualitative i.e. where a high accuracy of the level of enzymatic activity is not required, for example where it is suitable to test in there in no enzymatic activity, little enzymatic activity or high enzymatic activity.

Further, if only a minor amount of stabilizer is used it may not affect the enzymatic assay at all. The skilled person can by a few tests determine when removal of the stabilizer is desirable.

In an embodiment the stabilizer consist essentially of organic, polymer and up to about 5 % by weight of additive, such as surfactant(s) and/or dye(s), wherein the stabilizer is soluble in a liquid that does not dissolve or degrade the xerogel, preferably the stabilizer is soluble in an aqueous and/or alcoholic liquid, such as water, ethanol or a combination thereof.

In an embodiment the organic polymer of the stabilizer comprises at least one compound selected from vinyl containing compounds, such as polyvinylpyrrolidone, polyvinyl acetate, polyvinyl alcohol and copolymers comprising one or more of the mentioned vinyl compounds; acrylamide/acrylate based polymers or co-polymers thereof, such as octylacrylamide/acrylates/butylaminoethyl methacrylate copolymer and acrylates/T-butylacrylamide copolymer; silicone/siloxane-based polymers such as polydimethylsiloxane; polyethers such as polyethylene glycol; glycerol; ethylene glycol, propylene glycol, and/or any combinations thereof.

Advantageously a chemical stabilizer which has the properties of holding the substrate in place in the containing vessel and is easily washed away during an activation step is used.

The activation step comprises re-moistening the xerogel by a suitable liquid that does not degrade the xerogel e.g. as mentioned above. In general it is desired to use water or an aqueous buffer. Simultaneous, any surplus of dye may be removed.

The stabilizer(s) may be added by any method e.g. in form of solutions/mixtures with other solvents such as water or alcohols, or pure polymer. In an embodiment the stabilizer is mixed with the biopolymers prior to during or after cross-linking, such as before drying. Advantageously the stabilizer is sprayed onto the dry xerogel.

It has been found that where the stabilizer is mixed with the biopolymers prior to crosslinking, the stabilizer tends to block the filter where a filter is applied. Thus for the production of biopolymers substrates for assays using filters it is desirable that the stabilizer is added after the biopolymers have been crosslinked.

Further it has been found to be very effective and provide a preferred degree of mechanical stabilization to apply the stabilizer onto the biopolymer substrate after crosslinking and before drying.

In an embodiment the biopolymer substrate comprises up to 5 % by weight of stabilizer, such as from 0.01 to about 3 % by weight of stabilizer.

Advantageously the stabilizer is predominantly located on an outer surface area of the xerogel. Preferably the biopolymer substrate comprises an outer coating on at least a section of the biopolymer substrate.

In an embodiment the stabilizer is distributed onto the major part of the surface of the xerogel and optionally at least partly within the biopolymer substrate.

In an embodiment the stabilizer is distributed onto the surface area of the xerogel which is not in contact with a filter.

In an embodiment the stabilizer is distributed onto the surface area of the xerogel which is not in contact with the solid support.

In an embodiment the stabilizer is distributed onto the major part of the otherwise exposed surface of the xerogel i.e. the surface of the xerogel which without the stabilizer would have been exposed.

In an embodiment solid support structure is a well plate comprising at least one well. Such solid support structure is in the following referred to as a well plate. The well plate may in principle be any kind of well plate with any number and size of well(s). Advantageously the well plate is a multi-well plate comprising a plurality of wells.

In an embodiment the multi-well plate is a micro well plate, preferably comprising up to about 1536 wells. The term "micro well plate" is herein used to designate well plates comprising at least one well having a well with a volume less than about 10 ml, such as less than about 1 ml.

A suitable microplate advantageously has 6, 24, 96, 384 or even 1536 sample wells spatially arranged in row and column order. The wells may have any shape such as round or square.

In an embodiment the multi-well plate comprises one or more wells with a volume of up to about 25 ml or even more.

Preferably the multi-well plate comprises at least three wells, such as at least 6 wells. Preferably at least 2 wells comprise identical enzyme degradable biopolymer substrate that may be equal or different with respect to dye.

In an embodiment a biopolymer substrate is dyed with two dyes where the dyes advantageously have different properties or color (for example one is fluorescent, the other one is not). This enables measurement of activity of an enzyme on 2 different levels simultaneously. For example - one dye indicates an overall enzymatic activity and the other one can be applied in an additional step comprising electrophoresis for separating the soluble dyed oligomers e.g. using polyacrylamide carbohydrate electrophoresis (PACE).

In an embodiment the support structure comprises at least two wells comprising different enzyme degradable biopolymer substrates, preferably the support structure comprises at least three different enzyme degradable biopolymer substrates.

By applying equal and/or different biopolymer substrates in the various well of the multi-well plate the enzyme activity device provides a very beneficial device for use in high through put enzyme activity assays, which device is simple and fast to use, while simultaneously resulting in very accurate measurement(s) of enzymatic activity of a sample. The enzyme activity device comprising a multi-well plate solid support structure provides basis for providing a plurality of selected assays simultaneously while simultaneously obtaining high quality (with high accuracy) measurements for each well.

As mention above the respective biopolymer substrate in the respective wells may be equal or different, may comprise equal or different dye or dyes.

The amount of biopolymer substrate in the respective wells may be equal or different. In an embodiment the amount in weight of the biopolymer substrate in at least two wells with substantially identical enzyme degradable biopolymer substrate are substantially equal.

The amount of biopolymer substrate is selected in dependence on the type of assay to be performed and the amount of sample available. The amount of biopolymer substrate and the amount of sample is preferable adapted such that the major part of the biopolymer substrate can be wetted by the sample. The skilled person can be a few experiment find a suitable correlation of the amount of biopolymer substrate and the amount of sample.

In an embodiment amount of biopolymer substrate in each well is from about 1 mg to about 25 g, such as from about 5 mg to about 1 g, such as less than 50 mg.

In an embodiment the well plate is a filter well plate comprising filter means. The filter means may in principle be any kind of filter means suitable for filtering of the possibly degraded biopolymer substrate after a selected incubating time with a sample. For optimal filtering of degraded parts of the biopolymer substrate it is advantageous to rinsing with additional liquid, such as water or buffer and withdrawing the liquid through the filter means.

In an embodiment the filter means is arranged such that liquid and preferably optional fragments degraded from the biopolymer substrate and dispersed or dissolved by the liquid can pass through the filter means. The filer means is preferably operable by suction, however embodiments where the filter means is operable by pressure or merely gravity are not excluded.

Advantageously the filter means is provided by a filter structure for each well, which filter structure is arranged in a section of the well, preferably the filter structure of each well is arranged in a bottom section of the well.

Micro well plates (e.g. in form of micro titer plates) comprising a suitable filter means are well known and may e.g. be employed in the invention.

Such as the filter-microplate is for example marketed by AHN Biotechnologie GMBH, Nordhausen, Germany.

Advantageously the filter means has a cut-off size sufficiently large to allow passage of dissolved or dispersed fragments degraded from the biopolymer substrate to pass while retaining non degraded biopolymer substrate in the well(s).

In an embodiment the filter means has a cut-off size of about 10 µm or less, such as of about 5 µm or less, preferably about 1 µm or less.

In a variation of the solid support in form of the well plate, the solid substrate may be in form of a tube - i.e. corresponding to a single well of a well plate.

In an embodiment the tube is a vessel type tube as described in any one of US 3,593,909; US 4,713,219; US 5,270,011; EP 2 965 816; US 8,540,948 US 2007/0128080 or US 6,783,025.

In an embodiment the tube is an Eppendorf format tube with e.g. with a volume between 200 microliters and 2 milliliters. Such a tube is often referred to as a PCR type tube because it is suitable for performing Polymerase Chain Reaction

The tube may advantageously be an Eppendorf format tube in the following referred to as a PCR tube. In an embodiment the tube is a PCR tube with a filter, such as a filter insert. PCR tubes are well known in the art and are for examples marketed by Eppendorf. The tube may advantageously comprise a main tube structure and a filter insert for being inserted into the main tube structure. The biopolymer substrate is arranged in the filter insert. The filter insert has a bottom part comprising a filter. The filter may be as described above for the well plate filter. The main tube structure advantageously has a top section and a bottom section where the filter is inserted into the top section such that the filter is above the bottom section and by centrifugation or suction (e.g. vacuum) liquid and preferably optional fragments degraded from the biopolymer substrate and dispersed or dissolved by the liquid can pass through the filter means to be collected in the bottom section of the main tube structure. The tube may advantageously have a lid for protecting against contaminants during optional incubation time.

In a second aspect the solid support structure in the form of a lateral flow device supporting the biopolymer substrate. In this aspect the biopolymer substrate may be as above or alternatively the biopolymer substrate is a water insoluble hydrogel or aerogel comprising a network of dyed and cross-linked biopolymers, where the biopolymers and the dyes advantageously is as described above.

In this second aspect the enzyme activity device for determination of biopolymer enzyme degrading activity in a liquid sample comprises the biopolymer substrate and the solid support structure in the form of the lateral flow device supporting the biopolymer substrate wherein the biopolymer substrate is a dyed and water insoluble hydrogel, aerogel or xerogel comprising a network of cross-linked biopolymers.

Lateral flow devices and the principle applied using lateral flow devices is well known to a skilled person and the skilled person can by trial and error find a suitable lateral flow device solid support structure.

Advantageously the lateral flow device comprises a rigid support carrying a sample pad and a membrane structure arranged to form a pathway, wherein the biopolymer substrate is arranged onto the sample pad.

The sample pad is advantageously substantially non-liquid absorbing, such that the sample pad does substantially nod absorb a sample applied onto the biopolymer substrate. Preferably the sample pad has a hydrophobic surface supporting the biopolymer substrate.

In an embodiment the sample pad has a non-porous surface supporting the biopolymer substrate. Preferably the sample pad is non-porous.

The purpose of the sample pad is to support the sample and the sample when applied, such that the sample is not sucked into the sample pad or in other way transported away from the biopolymer substrate.

In an embodiment the sample pad has a cavity containing the biopolymer substrate. The cavity is for example in form of a depression in the sample pad.

In an embodiment the biopolymer substrate is supported by the sample pad at a distance to the membrane structure. Thereby a sample added to the substrate is can be held away from the membrane structure for a selected incubating time.

In an embodiment the membrane structure is of one or more porous material, preferably comprising nitrocellulose.

Advantageously the membrane structure is arranged immediately adjacent to or overlapping with the sample pad, such than when adding addition fluid onto the biopolymer substrate after an incubating time, the liquid washes degraded fragments (if any) of the biopolymer substrate and dyes which is optionally bound to the fragments onto the membrane structure which carries the degraded fragments and dyes further in lateral direction.

The wherein membrane structure comprises a read-out site which preferably is for visually read out.

The read out side can in principle be positioned at any position of the membrane structure. In an embodiment the read-out site is a site of the membrane structure with a laterally distance to the sample pad supporting the biopolymer substrate of at least about 5 mm, such as at least about 1 cm, such as up to 10 cm or even longer if desired.

In an embodiment the lateral flow device comprises a lid for the rigid support membrane structure, wherein the lid comprises an access opening to feed liquid onto the biopolymer substrate. Preferably the lid is constructed to allow read-out from the read-out site. The read-out site is optionally in form of a transparent window in the lid or merely a through hole in the lid.

In an embodiment the rigid support of the lateral flow device carries a plurality of sample pad sections and a plurality of membrane structure sections arranged to form a plurality of substantially parallel pathways, wherein each sample pad section supports a biopolymer substrate. Thereby several assays can be performed simultaneously.

In a third aspect the solid support structure in the form of a microfluidic device supporting the biopolymer substrate. In this aspect the biopolymer substrate may be as above or alternatively the biopolymer substrate is a water insoluble hydrogel or aerogel comprising a network of dyed and cross-linked biopolymers, where the biopolymers and the dyes advantageously is as described above.

In this second aspect for determination of biopolymer enzyme degrading activity in a liquid sample, the enzyme activity device comprises the biopolymer substrate and the solid support structure in form of the microfluidic device supporting the biopolymer substrate, wherein the biopolymer substrate is a dyed and water insoluble hydrogel, aerogel or xerogel comprising a network of cross-linked biopolymers.

Microfluidic devices comprising one or more microfluidic pathways are well known in the art and also methods of producing such microfluidic devices are well known to the skilled person.

Advantageously the microfluidic device comprises at least one flow channel comprising an inlet for feeding fluid into the flow channel, the flow channel comprising a sampling site comprising the biopolymer substrate.

Advantageously the biopolymer substrate is at least temporally fixed in the sampling site of the flow channel, such than when adding a sample or additional liquid such as washing liquid the biopolymer substrate or at least non-degraded biopolymer substrate remain at the sampling site. Preferably the sampling site is a chamber, wherein the chamber has a larger cross sectional area than the flow channel adjacent to the sampling site.

In an embodiment the flow channel comprises a read-out site. Preferably the microfluidic device comprises a transparent wall at the read-out site. The transparent wall is transparent at least for the dye of the biopolymer substrate, such that a dye at the read-out site can be read out via the read-out site optically or visually.

Advantageously the read-out site is positioned distally to the sampling site - i.e. longer away from the inlet than the sampling site. In principle the read-out site can be positioned at any distance to the sampling site, such as from a few mm to many cm, e.g. from about 5 mm to about 10 cm or even larger distance.

In an embodiment the flow channel comprises a flow stop between the sampling site and the read out site, the flow stop preferably being arranged immediately adjacent to the sampling site.

A flow stop is well known within the art of microfluidic devices, and may for example be in form of a hydrophobic barrier, a sharp edge or similar means which stops an aqueous liquid from flowing by capillary forces.

In an embodiment the microfluidic device comprises a sink section in fluid connection with the flow channel, wherein the fluid connection to the flow channel is positioned distal to the read-out section of the flow channel. Preferably the sink section has a volume which is larger than the volume of the flow channel.

In an embodiment the micro fluidic device comprises a flexible wall section of the flow channel and/or of the sink section in fluid connection with the flow channel, the flexible wall section can be pressed into the flow channel and/or the sink to generate fluid flow in the flow channel, preferably the flexible wall returns to its initial position when pressure pressing the flexible foil into the flow channel and/or the sink is released.

In an embodiment the microfluidic device comprises a rigid substrate with a groove and a foil covering the groove and fixed to the rigid substrate to form the flow channel.

Advantageously the rigid substrate comprises a cavity, the cavity is covered by the foil to provide the sink section.

Preferably the foil is a flexible foil providing a flexible wall section of the flow channel and/or of the sink section in fluid connection with the flow channel. The flexible wall section can be depressed into the flow channel and/or the sink to generate fluid flow in the flow channel, preferably the flexible wall returns to its initial position when pressure depressing the flexible foil into the flow channel and/or the sink is released.

In an embodiment the microfluidic device comprises a plurality of flow channels each flow channel comprises a sampling site comprising a biopolymer substrate. Thereby several assays may be performed simultaneously.

The disclosure also comprises a high throughput enzyme activity assay system suitable for determination of biopolymer enzyme degrading activity in a liquid sample. The assay system comprises
- an enzyme activity device comprising a solid support structure in form of a well plate,
- a filtrate collector comprising cavities for collecting filtrate from the wells, and
- a spectroscope for reading collected filtrate.

The enzyme activity device is as describe above where the solid support structure is a well plate, preferably a multi well plate.

In an embodiment the system comprises a filter plate arranged to place onto of the well plate to cover top opening(s) of the well(s). The well plate may comprise filter means as described above or the filter well plate may be free filter means.

In an embodiment the system comprises a holder for holding the filtrate collector and the solid support structure and optionally the filter plate if any in position during filtration, such that filtrate from the wells will be collected in one or more cavities of the filtrate collector.

The invention also comprises a method of determining enzyme activity of a sample the method comprising
- providing an enzyme activity device as described above;
- applying a preselected amount of the sample onto the biopolymer substrate;
- allowing the sample and the biopolymer substrate an incubating time of at least about 5 seconds, preferably at least about 10 seconds, such as at least about 30 seconds, such as at least about 1 minute;
- observing if dye and/or biopolymer fragments carrying dye is released from the biopolymer substrate; and
- determining the enzyme activity.

The determination of the enzyme activity may be a qualitative determination or a quantitative determination. Advantageously the determination of the enzyme activity is a quantitative determination. The determination may be provided by a method comprising a calibration, multiplexing or any similar method of determining from an optically or visually read-out.

In the embodiment where the solid support structure of the enzyme activity device is a well plate, the method advantageously comprises moisturizing the biopolymer substrate prior to adding the sample. The biopolymer substrate is preferably moisture by adding an aqueous liquid, such as water optionally comprising buffer or other suitable additives e.g. surfactants and removing non-adsorbed moisture for example by suction through the filter. Optionally the biopolymer substrate is washed one or more time prior to adding the sample in order to remove excess dye.

In an embodiment where the solid support structure of the enzyme activity device is a well plate the method comprises subjecting the incubated sample and biopolymer substrate to a filtration separating non-degraded biopolymer substrate from a filtrate comprising liquid and dissolved or dispersed fragments degraded from the biopolymer substrate and/or optionally released dye.

Advantageously the observation if dye and/or biopolymer fragments carrying dye are released from the biopolymer substrate is performed optically, preferably using a spectroscope.

In the embodiment where the solid support structure of the enzyme activity device is a lateral flow device plate, the method comprises adding aqueous liquid to the incubated sample and biopolymer substrate, wherein the amount of added aqueous liquid is sufficient to ensure wetting of the membrane.

Advantageously, the observation if dye and/or biopolymer fragments carrying dye are released from the biopolymer substrate is performed by visually reading out at the read-out site.

In the embodiment where the solid support structure of the enzyme activity device is a microfluidic device and the preselected amount of the sample is fed into the flow channel via the inlet.

In an embodiment the sample is drawn into the flow channel and onto the biopolymer substrate partly or fully by capillary forces.

Advantageously the sample is sucked into the flow channel and onto the biopolymer substrate, preferably by depressing and releasing a flexible wall of the flow channel or the sink, optionally by manually pressing and releasing or by using an actuator.

In an embodiment the method comprises adding aqueous liquid via the inlet into the channel, preferably by suction to the incubated sample and biopolymer substrate, wherein the amount of added aqueous liquid and/or the suction is sufficient to ensure that liquid reaches the read-out section, thereby any dyed fragments of degraded biopolymer substrate or any released dye reaches the read-out section and can be read out by a user optically and/or visually.

All features of the inventions including ranges and preferred ranges can be combined in various ways within the scope of the invention, unless there are specific reasons for not to combine such features.

### BRIEF DESCRIPTION OF DRAWINGS AND EXAMPLES

The invention will be explained more fully below in connection with a preferred embodiment and with reference to the drawings in which:
Figs. 1a and 1b show an enzyme activity device of an embodiment of the invention comprising a solid support structure in form of a multi well plate.
Fig 2. Shows a cover for the solid support structure shown in figs. 1a/1b.
Fig. 3A shows an enzyme activity device of an embodiment of the invention comprising a solid support structure in form of a lateral flow device.
Fig. 3B shows the solid support structure of the enzyme activity device of fig. 3B.
Fig. 4 shows a test performed using the enzyme activity device of fig. 3A.
Fig. 5 shows an enzyme activity device of an embodiment of the invention comprising a solid support structure in form of a microfluidic device.
Fig. 6 shows an enzyme activity assay system of an embodiment of the disclosure.
Fig. 7a shows an enzyme activity device of an embodiment of the invention comprising a solid support structure in form of a tube.
Fig. 7b shows an enzyme activity device of an embodiment of the invention comprising a solid support structure in form of a tube with a filter.
Fig. 8 shows used enzyme activity devices of the enzyme activity device type shown in Fig. 7a.
Fig. 9 shows two enzyme activity devices of the enzyme activity device type shown in Fig. 7b before or after use.

The figures are schematic and may be simplified for clarity. Throughout, the same reference numerals are used for identical or corresponding parts.

It should be understood that the specific examples, while indicating preferred embodiments of the invention, are given by way of illustration only.

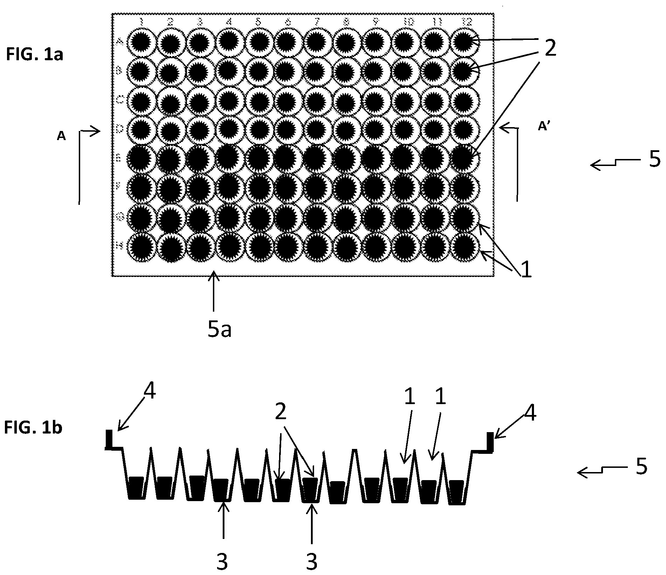

The enzyme activity device 5 shown in fig. 1a is seen in a top view and in fig. 1b the enzyme activity device is seen in a cross sectional side view in the line A-A' if fig. 1a.

The enzyme activity device 5 comprises a multi-well plate 5a comprising a plurality of wells 1 arranged in a matrix A-H x 1-12. The multi-well plate 5a comprises an edge 4 for holding it in a suitable holder. The wells 1, of the multi-well plate 5a has a bottom 3 which preferably comprises a filter constituting a filter means for separating liquid from un-degraded biopolymer substrate. Each well comprises a biopolymer substrate 2 comprising a dyed and water insoluble xerogel comprising a network of cross-linked biopolymers as described above. In the shown embodiment the biopolymer substrates 2 in the wells of rows A, B, C and D are smaller than the biopolymer substrates 2 in the rows E, F, G and H.

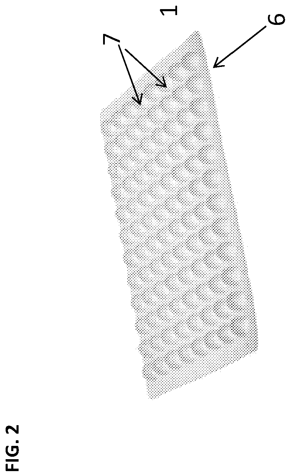

Fig. 2 shows a cover 6 suitable for the solid support structure 5 shown in figs. 1a/1b. The cover 6 comprises a plurality of convex protrusions arranged in a pattern corresponding to the wells. The cover is advantageously of a flexible material, such as rubber or silicone. In use the cover is arranged to cover the openings of the wells and a protrusion is inserted into each well. Thereby additional protection of the biopolymer substrate is provided during transport of the enzyme activity device. The cover 6 also shields the biopolymer substrate from being contaminated with dirt and etc.

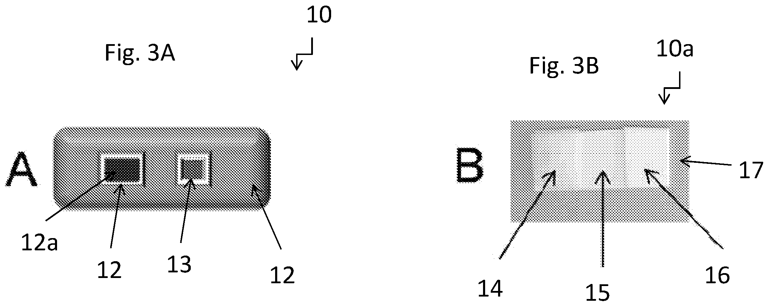

The enzyme activity device 10 of fig. 3a and fig. 3b is comprises a solid support structure in form of a lateral flow device . The enzyme activity device 10 comprises a lid 11 for the solid support structure 10a. The lid 11 comprises an access opening 12 to feed liquid onto a biopolymer substrate 12a. The lid further comprises a read-out window 13 allowing read-out from the read out site.

The solid support structure 10a comprises a rigid base 17, supporting a sample pad 14 and a membrane structure comprising a plurality of membrane structure pads 15, 16 of porous material. Preferably the membrane structure pads 15, 16 have different porosities such that liquid it travelling very fast in the first membrane structure pad 15 (smaller porosity), a where the travelling velocity in the second membrane structure pad 16 with larger porosity, is relatively low, while at the same time the second membrane structure pad 16 has a relatively large liquid capacity. The read-out site is in this embodiment at the second membrane structure pad 16.

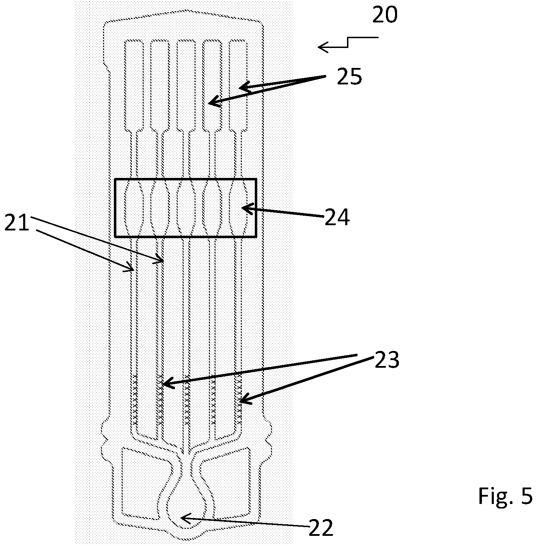

The enzyme activity device 20 of fig. 5 comprises a solid support structure in form of a microfluidic device comprising 5 flow channels 21 and a common inlet 22 for the flow channels 21. Each flow channel 21 comprises a sampling site comprising a biopolymer substrate 23. Each flow channel 21 comprises a read-out site 24 and is in fluid connection with a sink section 25. In the shown embodiment the read-out sites are enlarged to contain a relatively large amount of liquid compared with the amount of liquid which can be in the sampling site. Thereby it is easy to avoid that released dye /fragments with dye is washed into the sink section 25. In an alternative embodiment the read-out site has same cross-.sectional area as the sampling site.

In use the sample is added to the inlet 5 and by capillary forces it is brought into contact with the biopolymer substrates 23 in each channel 25. After a predetermined incubating time, water or buffer is sucked into the channels 21 by depressing a not shown film covering the sink sections 25 and if the biopolymer substrate has been degraded released dye or fragments comprising dye will be visible at the read-out sites 24.

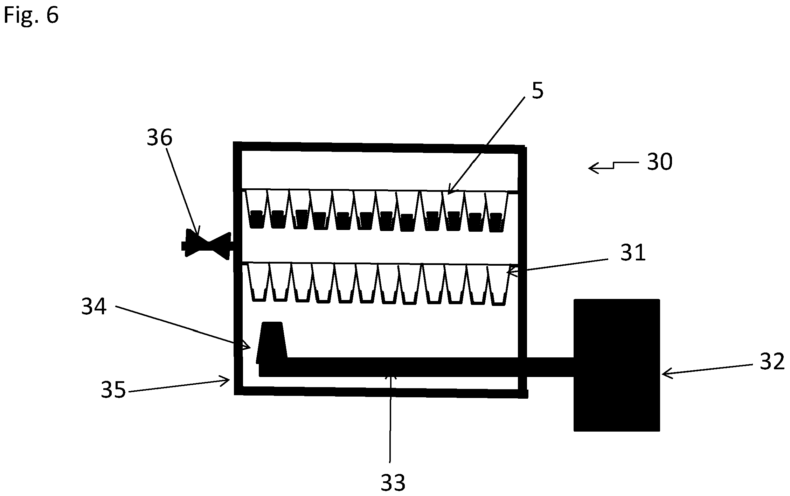

The enzyme activity assay system 30, shown in fig. 6 comprises an enzyme activity device 5 as shown in fig. 1a/1b and a filtrate collector 31 comprising cavities for collecting filtrate from the wells 1. The cavities of the filtrate collector 31 is arranged similar to the matrix arrangement of the wells of the enzyme activity device 5, thereby liquid from one well 1 will be collected in one selective cavity of the filtrate collector 31. The enzyme activity device 5 the filtrate collector 31 is mounted in a holder 35, such that filtrate passing the bottom 3 filtrate means of the enzyme activity device 5 will be collected in the respective cavities of the filtrate collector 31. A vacuum arrangement 36 is connected to the holder 35 to provide that the filtrate is sucked through the bottoms 3 of the wells.

A spectroscope 32 comprising a movable arm 33 and a spectroscope head for collecting light is arranged to measure the wavelengths emitted from or reflected from or transmitted through the collected filtrate in the respective cavities of the filtrate collector 31.

A not shown light source may advantageously be arranged to illuminating the collected filtrate.

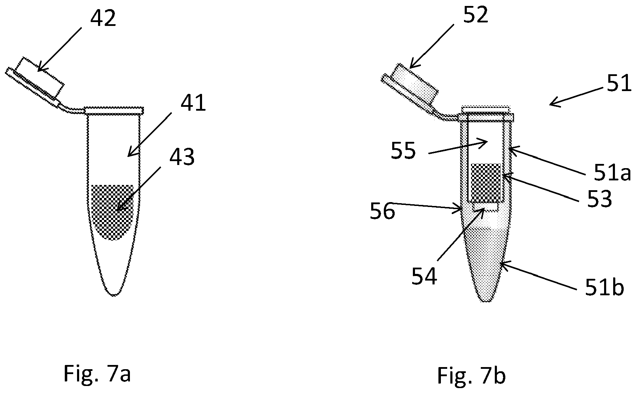

The enzyme activity device shown in fig. 7a comprises a solid support structure in form of a tube 41 with a lid 42 and a biopolymer substrate 43 as described above arranged within the tube

The enzyme activity device shown in fig. 7b comprises a solid support structure in form of a tube 51 with a lid 52. The tube 51 comprises a main tube structure 56 and an insert 55 with a filter 54 at its bottom part. The enzyme activity device further comprises a biopolymer 53 substrate as described above arranged within the insert 55. The main tube structure 56 has a top section and a bottom section where the filter is inserted into the top section such that the filter is above the bottom section.

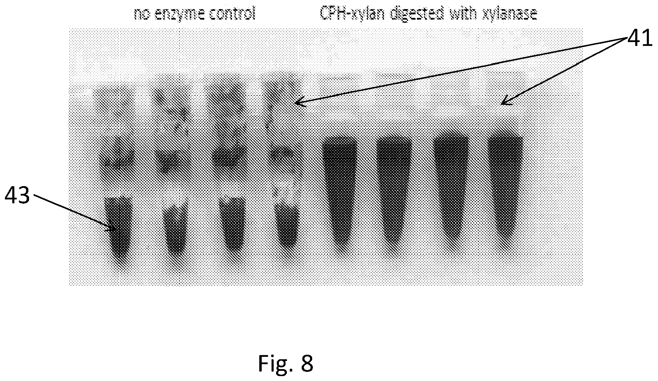

Fig. 8 shows a row of 8 used enzyme activity devices of the enzyme activity device type shown in fig. 7a. The substrate 43 used in these devices was a red dyed oligosaccharide biopolymer substrate which was degradable by xylanase. Liquid sample without active endo-xylanase was added to the 4 left enzyme activity devices and liquid sample with active endo-xylanase was added to the 4 left enzyme activity devices. The lids were closed and the enzyme activity devices were shaken manually for about 10 seconds. As seen the 4 left samples did not degrade the substrate - the substrate was merely mechanically damaged by the shaking. The 4 left samples, however showed clearly that the substrate was degraded and the dye comprising soluble or dispersible fragment of the biopolymer was evenly distributed in the sample fluid. The resulting degradation may be further examined by spectrometry as described above.

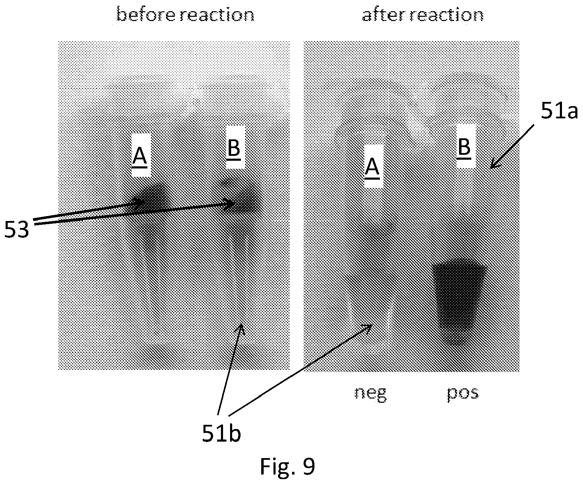

Fig. 9 shows two enzyme activity devices in the left image before use. The enzyme activity devices are of the type shown in fig. 7b i.e. a tube comprising a filter.

A sample with enzyme activity for the biopolymer substrate was added in one of the enzyme activity devices B and a sample without enzyme activity for the biopolymer substrate was added in one of the enzyme activity devices A. The two enzyme activity devices was centrifuged (spun) and a filtrate comprising optional dye or dye bond to biopolymer fragments was collected in the bottom section 51b of the respective enzyme activity devices A and B. It is clearly seen that there was no enzyme activity of the sample added to the biopolymer substrate of device A, whereas the sample added to the biopolymer substrate of device B had a high enzyme activity. The enzyme activity may be further quantified by spectrometry as described above.

### Example 1

### Product plate volume variation comparison using fresh non-dried hydrogel and freeze-dried xerogel substrates

According to the invention it has been found that using fresh non-dried hydrogel substrates, the hydrogel was found to absorb or comprise an excessive amount of water with a variable and unpredictable swelling degree By freeze-drying the hydrogel to obtain xerogel substrates according to an embodiment of the invention, the substrates do not swell back to their original volume when re-suspended in water/buffer.

An experiment with fresh (non-dried hydrogel) substrates and freeze-dried substrates according to an embodiment of the invention was conducted where the substrates were partially degraded and the recovery volumes of the product supernatants were measured.

The experiment was conducted with 12 different substrates.

Table 3 shows a statistical analysis of volume variation comparison between assays done with fresh and freeze-dried substrates

**TABLE 3**

| A | | MEAN | |
|---|---|---|---|
| | FRESH VOLUME WITHOUT ENZYME SEM | 2,5 | |
| | FREEZE-DRIED VOLUME WITHOUT ENZYME SEM | 1,2 | |
| | FRESH VOLUME WITH ENZYME SEM | 7,4 | |
| | FREEZE-DRIED VOLUME WITH ENZYME SEM | 1,0 | |
| | NO SUBSTRATE VOLUME SEM | 0,3 | |

| B | | MEAN | SEM |
|---|---|---|---|
| | VOLUME DEVIATION FRESH WITHOUT ENZYME % | 25,8 | 4,3 |
| | VOLUME DEVIATION FREEZE-DRIED WITHOUT ENZYME % | 8,1 | 3,2 |
| | VOLUME DEVIATION FRESH WITH ENZYME % | 25,6 | 4,5 |
| | VOLUME DEVIATION FREEZE-DRIED WITH ENZYME % | 9,3 | 3,1 |
| | NO SUBSTRATE VOLUME DEVIATION % | 0,9 | 0,7 |

The analysis of the average standard error of means (SEM) in Table 1 (panel A) shows that the values for freeze dried substrates without (1.2 µL) and with the enzyme (1.0 µm) are lower than the values of the same settings with fresh substrates (2.5 µL and 7.4 µL, respectively).

To see how this beneficial effect of freeze-drying is beneficial for the assay, a volume deviation analysis was done where the deviation of the recovered volume from the added volume was statistically analyzed (Table 3, panel B). It is observed that the mean values for the fresh substrates without and with enzyme treatment (25.8% and 25.6%) are significantly higher than those for the freeze-dried substrates (8.1% and 9.3%, respectively).

The "No substrate" value takes into account the amount of liquid lost if just the liquid is added to an empty well and then filtered into a collection plate. The difference between the added volume and the recovered volume is the "No substrate" value.

### Example 2

### RE-SWELLING AFTER FREEZE-DRYING

12 different hydrogels of cross-linked biopolymers was prepared. The hydrogels are referred to as CPH-X where X indicate the cross-linked biopolymer obtained from natural sources corresponding to the sources listed I table 1 (yellow CPH-casein, yellow CPH-pectic galactan, yellow CPH-lichenan, yellow CPH-arabinoxylan, yellow CPH-amylose, yellow CPH-galactomannan, blue CPH dextran, blue CPH-arabinan, blue CPH-beta-glucan (barley), blue CPH-pectic galactan, blue CPH-amylopectin, blue CPH-arabinoxylan). The CPH substrates were transferred into a 96 well filter plate (8 replicas per CPH substrate). Additional water was removed by centrifugation. The plate containing the CPH substrate was frozen at -80 °C and then freeze dried.

There was no preservative/stabilizer added in this measurement, because we didn't want to confound the data with added mass from an additional compound.

| | |
|---|---|
| No substrates | 51.35 g |
| Weight of the plate with substrate | 66.12 g |
| After centrifugation (10 min at full speed) | 59.48 g |
| Weight of the plate after freeze drying | 51.59 g |
| Activation (adding 200 µl water to each well) for 25 min | 70.50 g |
| After vacuum filtration | 56.23 g |
| After centrifugation (2 min at full speed) | 55.26 g |

To better explain the data, the weight of an empty filter plate is 51.35 g and after the fresh substrates have been added it weighed 66.12 g (so that's additional 14.77 g and that's the weight of 96 wells full of substrates). Extra water was removed by centrifugation leaving 59.48 g meaning that the weight of the fresh non-dried hydrogel substrates without additional water is 8.13 g.

After freeze-drying, the weight of the substrate plate was reduced to 51,59 g meaning that the weight of the substrates when dry is 0.24 g.

Water was added to the dried substrates, incubated for 25 minutes and then removed by vacuum filtration to yield a weight of 56.23 g meaning that the substrates have absorbed 4.64 g of water.

By centrifugation, some additional water could be removed from the re-swollen substrates (referred to as CPX-substrates) yielding 3.67 g of re-swollen substrate.

By analyzing these measurements, we found:
- Fresh substrate in fact contains only 3% dry weight of substrate, the rest is water
- When freeze-dried and re-swollen for 25 minutes in deionized water - the substrate re-swells only to 45% of its original water content

### Example 3

### Lateral flow device

An the enzyme activity device comprising a solid support structure in form of a lateral flow device as shown in figs. 3A and 3B was provided. The biopolymer substrate 12a supported on the sample pad 14 was a CPX-xylan substrate.

A liquid sample comprising the corresponding enzyme xylanase was added to the biopolymer substrate 12a and was allowed 5 minutes incubation time.

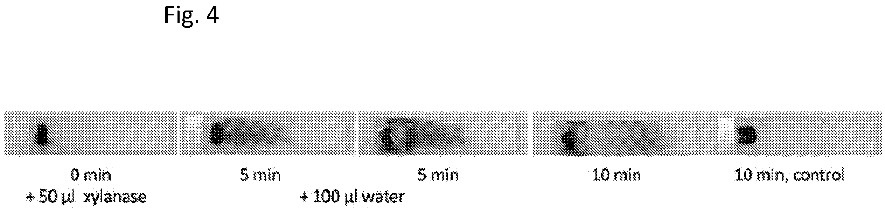

During the incubating time the xylanase degraded the substrate CPx-xylan substrate and produced a coloured solution, which started to migrate along the first the membrane structure pad 15 as shown in fig. 4. Thereafter 100 µl water was added onto the biopolymer substrate 12a and the colored supernatant (reaction product) is moving towards the second membrane structure pad 16. After 10 minutes the second membrane pad 16 comprising the read-out site, is colored, which will be visible via the read out window 13). If the substrate is not degraded by the enzyme, no colored supernatant is released and the membrane remains colorless as shown in the control.

### (Control after 10 min, fig. 4).

### Example 4

### Production of CPX substrate with stabilizer

The biopolymer xyloglucan is obtained from tamarind. Such biopolymer can for example be purchased from Megazyme International, Ireland: The biopolymer is dissolved in aqueous sodium hydroxide (0.5M sodium hydroxide) at room temperature or heating at 65 °C if needed for fully dissolving. For increasing dissolution, it is desired to subject it to shaking at 120 rpm. After complete dissolution, the dye is added. Different dyes can be selected (examples: reactive red 4, reactive green 19, reactive blue 49, reactive yellow 2) and the solvation is incubated for 30 minutes at room temperature with shaking at 120 rpm.

After that, the cross linker is added (1,4-butanediol diglycidyl ether), the reaction mixture is briefly vortexed and the reaction mixture is left to stand for 3 days and during that time - an aerogel forms.

The aerogel is then homogenized for example using a spatula to thereby split the hydrogel. The desired hydrogen particle size can be selected to the skilled person. By splitting the hydrogel a more uniform pore size of the final xerogel can be obtained.

Thereafter the hydrogel is washed with hot water (80-100 °C) until all of the excess dye is washed away. The aerogel is then stored at +4 °C.

The aerogel is transferred to the wells (100-150 µl per well) of a 96 well filter plate using a pipette and 15 µl stabilizer is added on top of each aerogel well portion. The stabilizer comprises 0.5% PVP-360 and/or 1% PVP-40. The plate is then frozen at -80 °C for at least 30 min and freeze dried overnight.

It will be seen that a very stable and mechanical strong xerogel biopolymer substrate is obtained. The stabilizer will ensure that the biopolymer substrate is not damaged during transport of the t enzyme activity device.

### Example 5

### Use of the CPX substrate with stabilizer

The plate containing the dried CPX substrate can be stored at room temperature until use.

In use the CPX substrate is activated by adding 200 µl water to each well and allow incubating for 15 min. The dry CPX substrate will be restored to a hydrogel but with a different pore structure than before the drying as it is explained above. The remaining water is removed by vacuum filtration or centrifugation. After that, the substrate is washed twice with 100 µl water to remove the stabilizer.

The experiment is performed by adding the sample in an appropriate buffer (between pH 3.0-10.0) with a total volume between 150-200 µl. To avoid undesired evaporation a plastic lid can be added on top of the well prior to incubating at a predetermined time (between 10 min - 24 h) and temperature (up to 90 °C). The plate is advantageously be shaken (around 150 rpm) during the reaction. The reaction is stopped by separating the supernatant from the non-degraded substrate (using vacuum filtration or centrifugation). The filtrate is collected in an Elisa plate (filtrate collector) and quantified using a spectrophotometer.

### TABLES

**Table 1. Examples of CPX substrates suitable for embodiments of the enzyme activity device of the invention.**

| **Substrate** | **Source** |
|---|---|
| CPX-2-hydroxyethylcellulose (CPX-HE cellulose) | N/A |
| CPX-amylopectin | potato |
| CPX-amylose | potato |
| CPX-arabinan | sugar beet |
| CPX-arabinoxylan | wheat |
| CPX-casein | bovine milk |
| CPX-curdlan | *Alcaligenes faecalis* |
| CPX-dextran | *Leuconostoc spp.* |
| CPX-galactomannan | carob |
| CPX-laminarin | *Laminaria digitata* |
| CPX-lichenan | Icelandic moss |
| CPX-pachyman | *Poria cocos* |
| CPX-pectic galactan | potato |
| CPX-pullulan | *Aureobasidium pullulans* |
| CPX-rhamnogalacturonan I | potato |
| CPX-rhamnogalacturonan I (-Gal) 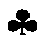 | potato |
| CPX-rhamnogalacturonan | soy bean |
| CPX-xylan | beechwood |
| CPX-xyloglucan | tamarind |
| CPX-β-glucan from barley | barley |
| CPX-β-glucan from oat | oat |
| CPX-β-glucan from yeast | yeast |

| | |
|---|---|
| 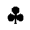 (β-1,4-D-galactan side chains removed with endo-galactanase *gal*) | |

**Table 2. Examples of enzymes that can be tested for using an enzyme activity device within the scope of the invention including code, source and Carbohydrate Active Enzyme database (CAZy) family**

| **Code name** | **Description** | **CAZy family** | **Source** |
|---|---|---|---|
| *ara* | Endo-arabinase (*Aspergillus niger*) | GH43 | Megazyme |
| *cell* | Endo-cellulase (endo-β-1,4-glucanase) (*Trichoderma longibrachiatum*) | GH7 | Megazyme |
| *cel2* | Cellulase (endo-β-1,4-glucanase) (*Bacillus amyloliquefaciens*) | GH5 | Megazyme |
| *Gal* | Endo-β-1,4-D-galactanase *(Aspergillus niger)* | GH53 | Megazyme |
| *glc* | Endo-β-1,3-glucanase (*Trichoderma sp.*) | GH16 | Megazyme |
| *lic* | Lichenase (endo-β-1,3(4)-glucanase) (*Bacillius sp*.) | GH16 | Megazyme |
| *man* | Endo-β-1,4-mannanase (*Cellvibrio japonicus*) | GH26 | Megazyme |
| nz1 | Endo-β-1,4-xylanase (*Aspergillus aculeatus*) | GH10 | Novozymes |
| nz2 | Endo-β-1,4-xylanase (*Thermomyces lanuginosus*) | GH11 | Novozymes |
| nz3 | Endo-β-1,4-glucanase *(Aspergillus aculeatus*) | GH5 | Novozymes |
| nz4 | Proprietary fungal endo-β-1,4-mannanase | GH5 | Novozymes |
| *ply1* | Macerase™ Pectinase (*Rhizopus sp*.) | N/A | Calbiochem |
| *ply2* | Pectolyase Y-23 (*Aspergillus japonicus*) | N/A | Duchefa Biochemie |
| *ply3* | Pectolyase (*Aspergillus japonicus*) | N/A | Sigma |
| *pec1* | Pectate lyase (*Cellvibrio japonicus*) | PL10 | Megazyme |
| *pec2* | Pectate lyase (*Aspergillus sp*.) | N/A | Megazyme |
| *pol1* | Endo-polygalacturonase M2 (*Aspergillus niger*) | GH28 | Megazyme |
| *pol2* | Endo-polygalacturonase M1 (*Aspergillus niger*) | GH28 | Megazyme |
| *rgh* | Rhamnogalacturonan hydrolase (*Aspergillus aculeatus*) | GH28 | Novozymes |
| *xg* | Xyloglucanase (*Paenibacillus sp.*) | GH5 | Megazyme |
| *xyl1* | β-xylanase, M4 (*Aspergillus niger*) | GH11 | Megazyme |
| *xyl2* | Endo-β-1,4-xylanase M1 (*Trichoderma viride*) | GH11 | Megazyme |
| *Nc*LPMO9C | Lytic polysaccharide monoxygenase (*Neurospora crassa*) | AA9 | N/A |
| Broth | Culture broth from *Phanerochaete chrysosporium* (3d after inoculation) | N/A | N/A |
| Proteinase K | Proteinase K (*Tritirachium album*) | N/A | Sigma |
| Trypsin | Trypsin from bovine pancreas | N/A | Sigma |
| Elastase | Elastase from porcine pancreas | N/A | Sigma |

Some preferred embodiments have been shown in the foregoing, but it should be stressed that the invention is not limited to these, but may be embodied in other ways within the subject-matter defined in the following claims.

## Claims

1. An enzyme activity device suitably for determination of biopolymer enzyme degrading activity in a liquid sample, said enzyme activity device comprising a biopolymer substrate and a solid support structure supporting said biopolymer substrate wherein said biopolymer substrate comprises a water insoluble xerogel comprising a network of cross-linked biopolymers and wherein the biopolymer substrate comprises a dye chemically bound to the biopolymer.

2. The enzyme activity device of claim 1, wherein the xerogel has a density of from about 0.02 g/cm³ to about 0.5 g/cm³.

3. The enzyme activity device of claim 1 or claim 2, wherein the solid support structure is selected from a well containing test plate, a tube, a lateral flow device or a microfluidic device.

4. The enzyme activity device of any one of the preceding claims wherein the biopolymer substrate comprises cross-linked polymeric biomolecules selected from polynucleotides, polypeptides, polysaccharides or any combinations thereof.

5. The enzyme activity device of any one of the preceding claims, wherein the biopolymers comprises a plurality of enzyme degradable bonds, preferably at least about 50 % of said enzyme degradable bonds are degradable by identical enzymes.

6. The enzyme activity device of any one of the preceding claims, wherein the biopolymers comprises a plurality of enzyme degradable bonds, preferably at least about 75 % are exclusively enzyme degradable by one type of enzyme.

7. The enzyme activity device of any one of the preceding claims, wherein the xerogel is obtainable from a process comprising crosslinking and dying a plurality of polymeric biomolecules in an aqueous fluid to form a hydrogel, freezing the hydrogel to a temperature of less than about -25 °C and freeze-drying the hydrogel.

8. The enzyme activity device of any one of the preceding claims, wherein the xerogel has a porosity (gas fraction) of from about 20 % to about 85 %.

9. The enzyme activity device of any one of the preceding claims, wherein the xerogel has a volume, which is about 90 % or less relative to the volume of the gel from which the xerogel is obtained by drying.

10. The enzyme activity device of any one of the preceding claims, wherein the xerogel has a surface area of at least about 100 m²/g.

11. The enzyme activity device of any one of the preceding claims, wherein the biopolymer substrate comprises a stabilizer for stabilizing the xerogel, said stabilizer comprises at least one organic polymer and being soluble in a liquid that does substantially not dissolve or degrade the xerogel.

12. The enzyme activity device of claim 11, wherein the stabilizer is soluble in an aqueous and/or alcoholic liquid, such as water, ethanol or a combination thereof.

13. The enzyme activity device of any one of claims 11-12, wherein the stabilizer is predominantly located on an outer surface area of the xerogel.

14. The enzyme activity device of any one of the preceding claims 3-13, wherein the well containing test plate is a filter well plate comprising filter means, the filter means is arranged such that liquid and preferably optional fragments degraded from the biopolymer substrate and dispersed or dissolved by the liquid can pass through the filter means.

15. The enzyme activity device in accordance to any one of claims 3-13, wherein the solid support structure is in the form of a tube, the tube comprises a filter, preferably the tube comprises a main tube structure and a filter insert for being inserted into the main tube structure.

16. The enzyme activity device in accordance to any one of claims 3-13, wherein the solid support structure is in the form of a lateral flow device supporting said biopolymer substrate, and wherein the lateral flow device comprises a rigid support carrying a sample pad and a membrane structure arranged to form a pathway, wherein said biopolymer substrate is arranged onto said sample pad, said sample pad is substantially non-liquid absorbing, preferably the sample pad has a hydrophobic surface supporting the biopolymer substrate.

17. The enzyme activity device in accordance to any one of claims 3-13, wherein the solid support structure is in the form of a microfluidic device supporting said biopolymer substrate, wherein the microfluidic device comprises at least one flow channel comprising an inlet for feeding fluid into the flow channel, said flow channel comprising a sampling site comprising said biopolymer substrate.

18. A method of determining enzyme activity of a sample the method comprising
• providing an enzyme activity device comprising a biopolymer substrate and a solid support structure supporting said biopolymer substrate wherein said biopolymer substrate comprises a dyed and water insoluble xerogel comprising a network of cross-linked biopolymers;
• applying a preselected amount of the sample onto the biopolymer substrate;
• allowing the sample and the biopolymer substrate an incubating time of at least about 5 seconds, preferably at least about 10 seconds, such as at least about 30 seconds, such as at least about 1 minute;
• observing if dye and/or biopolymer fragments carrying dye is released from the biopolymer substrate; and
• determining the enzyme activity.

## Patentansprüche

1. Vorrichtung für Enzymaktivität, die zur Bestimmung der Biopolymer-abbauenden Enzymaktivität in einer flüssigen Probe geeignet ist, wobei die Vorrichtung für Enzymaktivität ein Biopolymersubstrat und eine feste Trägerstruktur, welche das Biopolymersubstrat trägt, umfasst, wobei das Biopolymersubstrat ein wasserunlösliches Xerogel umfasst, das ein Netzwerk von vernetzten Biopolymeren umfasst, und wobei das Biopolymersubstrat einen Farbstoff umfasst, der chemisch an das Biopolymer gebunden ist.

2. Vorrichtung für Enzymaktivität nach Anspruch 1, wobei das Xerogel eine Dichte von ungefähr 0,02 g/cm³ bis ungefähr 0,5 g/cm³ aufweist.

3. Vorrichtung für Enzymaktivität nach Anspruch 1 oder Anspruch 2, wobei die feste Trägerstruktur aus einer eine Vertiefung enthaltenden Testplatte, einem Röhrchen, einer Lateralfluss-Vorrichtung oder einer mikrofluidischen Vorrichtung ausgewählt ist.

4. Vorrichtung für Enzymaktivität nach einem der vorhergehenden Ansprüche, wobei das Biopolymersubstrat vernetzte polymere Biomoleküle umfasst, die aus Polynukleotiden, Polypeptiden, Polysacchariden oder beliebigen Kombinationen davon ausgewählt sind.

5. Vorrichtung für Enzymaktivität nach einem der vorhergehenden Ansprüche, wobei die Biopolymere eine Vielzahl von durch Enzyme abbaubaren Bindungen umfassen, wobei vorzugsweise mindestens ungefähr 50 % der durch Enzyme abbaubaren Bindungen durch identische Enzyme abbaubar sind.

6. Vorrichtung für Enzymaktivität nach einem der vorhergehenden Ansprüche, wobei die Biopolymere eine Vielzahl von durch Enzyme abbaubaren Bindungen umfassen, wobei vorzugsweise mindestens ungefähr 75 % ausschließlich durch einen Enzymtyp enzymatisch abbaubar sind.

7. Vorrichtung für Enzymaktivität nach einem der vorhergehenden Ansprüche, wobei das Xerogel durch ein Verfahren erhältlich ist, welches das Vernetzen und Färben einer Vielzahl von polymeren Biomolekülen in einem wässrigen Fluid unter Bildung eines Hydrogels, das Einfrieren des Hydrogels auf eine Temperatur von weniger als ungefähr -25 °C und das Gefriertrocknen des Hydrogels umfasst.

8. Vorrichtung für Enzymaktivität nach einem der vorhergehenden Ansprüche, wobei das Xerogel eine Porosität (Gasfraktion) von ungefähr 20 % bis ungefähr 85 % aufweist.

9. Vorrichtung für Enzymaktivität nach einem der vorhergehenden Ansprüche, wobei das Xerogel ein Volumen aufweist, das ungefähr 90 % oder weniger im Verhältnis zu dem Volumen des Gels beträgt, aus dem das Xerogel durch Trocknen erhalten wird.

10. Vorrichtung für Enzymaktivität nach einem der vorhergehenden Ansprüche, wobei das Xerogel einen Oberflächenareal von mindestens ungefähr 100 m²/g aufweist.

11. Vorrichtung für Enzymaktivität nach einem der vorhergehenden Ansprüche, wobei das Biopolymersubstrat einen Stabilisator zur Stabilisierung des Xerogels umfasst, wobei der Stabilisator mindestens ein organisches Polymer umfasst und in einer Flüssigkeit löslich ist, die das Xerogel im Wesentlichen nicht auflöst oder abbaut.

12. Vorrichtung für Enzymaktivität nach Anspruch 11, wobei der Stabilisator in einer wässrigen und/oder alkoholischen Flüssigkeit, wie etwa Wasser, Ethanol oder einer Kombination davon, löslich ist.

13. Vorrichtung für Enzymaktivität nach einem der Ansprüche 11 bis 12, wobei der Stabilisator hauptsächlich auf einem äußeren Oberflächenbereich des Xerogels angeordnet ist.

14. Vorrichtung für Enzymaktivität nach einem der vorhergehenden Ansprüche 3 bis 13, wobei die eine Vertiefung enthaltende Testplatte eine Filtervertiefungsplatte ist, welche eine Filtereinrichtung umfasst, wobei die Filtereinrichtung derart angeordnet ist, dass Flüssigkeit und vorzugsweise optionale Fragmente, welche von dem Biopolymersubstrat abgebaut und durch die Flüssigkeit dispergiert oder gelöst werden, durch die Filtereinrichtung hindurchgehen können.

15. Vorrichtung für Enzymaktivität nach einem der Ansprüche 3 bis 13, wobei die feste Trägerstruktur in der Form eines Röhrchens vorliegt, wobei das Röhrchen einen Filter umfasst, wobei vorzugsweise das Röhrchen eine Hauptröhrchenstruktur und einen Filtereinsatz umfasst, der zum Einsetzen in die Hauptröhrchenstruktur ist.

16. Vorrichtung für Enzymaktivität nach einem der Ansprüche 3 bis 13, wobei die feste Trägerstruktur in der Form einer Lateralfluss-Vorrichtung vorliegt, die das Biopolymersubstrat trägt, und wobei die Lateralfluss-Vorrichtung einen starren Träger umfasst, der ein Probenkissen und eine Membranstruktur trägt, welche so angeordnet sind, dass sie einen Pfad bilden, wobei das Biopolymersubstrat auf dem Probenkissen angeordnet ist, wobei das Probenkissen im Wesentlichen nicht flüssigkeitsabsorbierend ist, wobei das Probenkissen vorzugsweise eine hydrophobe Oberfläche aufweist, die das Biopolymersubstrat trägt.

17. Vorrichtung für Enzymaktivität nach einem der Ansprüche 3 bis 13, wobei die feste Trägerstruktur in der Form einer mikrofluidischen Vorrichtung vorliegt, die das Biopolymersubstrat trägt, wobei die mikrofluidische Vorrichtung mindestens einen Strömungskanal umfasst, der einen Einlass zum Zuführen von Fluid in den Strömungskanal umfasst, wobei der Strömungskanal eine Beprobungsstelle umfasst, die das Biopolymersubstrat umfasst.

18. Verfahren zur Bestimmung der Enzymaktivität einer Probe, wobei das Verfahren umfasst
• das Bereitstellen einer Vorrichtung für Enzymaktivität, die ein Biopolymersubstrat und eine feste Trägerstruktur umfasst, die das Biopolymersubstrat trägt, wobei das Biopolymersubstrat ein gefärbtes und wasserunlösliches Xerogel umfasst, das ein Netzwerk aus vernetzten Biopolymeren umfasst;
• das Aufbringen einer vorgewählten Menge der Probe auf das Biopolymersubstrat;
• das Ermöglichen einer Inkubationszeit der Probe und des Biopolymersubstrats von mindestens ungefähr 5 Sekunden, vorzugsweise mindestens ungefähr 10 Sekunden, wie etwa mindestens ungefähr 30 Sekunden, wie etwa mindestens ungefähr 1 Minute;
• das Beobachten, ob Farbstoff und/oder farbstofftragende Biopolymerfragmente von dem Biopolymersubstrat freigesetzt werden; und
• das Bestimmen der Enzymaktivität.

## Revendications

1. Dispositif de dosage d'activité enzymatique permettant de déterminer l'activité de dégradation enzymatique d'un biopolymère dans un échantillon liquide, ledit dispositif de dosage d'activité enzymatique comprenant un substrat biopolymère et une structure de support solide supportant ledit substrat biopolymère dans lequel ledit substrat biopolymère comprend un xérogel insoluble dans l'eau comprenant un réseau de biopolymères réticulés et dans lequel le substrat biopolymère comprend un colorant chimiquement lié au biopolymère.

2. Dispositif de dosage de l'activité enzymatique selon la revendication 1, dans lequel le xérogel a une densité d'environ 0,02 g/cm³ à environ 0,5 g/cm³.

3. Dispositif de dosage de l'activité enzymatique selon la revendication 1 ou la revendication 2, dans lequel la structure de support solide est sélectionnée parmi une plaque à puits de test, une éprouvette, un dispositif à écoulement latéral ou un dispositif microfluidique.

4. Dispositif de dosage de l'activité enzymatique selon l'une quelconque des revendications précédentes dans lequel le substrat biopolymère comprend des biomolécules polymères réticulées sélectionnées parmi des polynucléotides, des polypeptides, des polysaccharides ou toute combinaison de ceux-ci.

5. Dispositif de dosage de l'activité enzymatique selon l'une quelconque des revendications précédentes, dans lequel les biopolymères comprennent une pluralité de liaisons dégradables par voie enzymatique, de préférence au moins environ 50 % desdites liaisons dégradables par voie enzymatique sont dégradables par des enzymes identiques.

6. Dispositif de dosage de l'activité enzymatique selon l'une quelconque des revendications précédentes, dans lequel les biopolymères comprennent une pluralité de liaisons dégradables par voie enzymatique, de préférence au moins environ 75 % sont dégradables par voie enzymatique exclusivement par un type d'enzyme.

7. Dispositif de dosage de l'activité enzymatique selon l'une quelconque des revendications précédentes, dans lequel le xérogel peut être obtenu par un procédé comprenant la réticulation et la coloration d'une pluralité de biomolécules polymères dans un fluide aqueux pour former un hydrogel, la congélation de l'hydrogel à une température inférieure à environ -25 °C et lyophilisation de l'hydrogel.

8. Dispositif de dosage de l'activité enzymatique selon l'une quelconque des revendications précédentes, dans lequel le xérogel a une porosité (fraction de gaz) d'environ 20 % à environ 85 %.

9. Dispositif de dosage de l'activité enzymatique selon l'une quelconque des revendications précédentes, dans lequel le xérogel a un volume qui est d'environ 90 % ou moins par rapport au volume du gel à partir duquel le xérogel est obtenu par séchage.

10. Dispositif de dosage de l'activité enzymatique selon l'une quelconque des revendications précédentes, dans lequel le xérogel a une aire de surface d'au moins environ 100 m²/g.

11. Dispositif de dosage de l'activité enzymatique selon l'une quelconque des revendications précédentes, dans lequel le substrat biopolymère comprend un stabilisant pour stabiliser le xérogel, ledit stabilisant comprend au moins un polymère organique et étant soluble dans un liquide qui ne dissous ou dégrade pas sensiblement le xérogel.

12. Dispositif de dosage de l'activité enzymatique selon la revendication 11, dans lequel le stabilisant est soluble dans un liquide aqueux et/ou alcoolique, tel que l'eau, l'éthanol ou une combinaison de ceux-ci.

13. Dispositif de dosage de l'activité enzymatique selon l'une quelconque des revendications 11 et 12, dans lequel le stabilisant est principalement situé sur une aire de surface extérieure du xérogel.

14. Dispositif de dosage de l'activité enzymatique selon l'une quelconque des revendications précédentes 3 à 13, dans lequel la plaque à puits de test est une plaque à puits de filtration comprenant un moyen de filtration, le moyen de filtration est agencé de telle sorte que le liquide et de préférence les fragments facultatifs dégradés du substrat biopolymère et dispersés ou dissous par le liquide peuvent passer à travers le moyen de filtration.

15. Dispositif de dosage de l'activité enzymatique selon l'une quelconque des revendications 3 à 13, dans lequel la structure de support solide se trouve sous la forme d'une éprouvette, l'éprouvette comprend un filtre, de préférence l'éprouvette comprend une structure d'éprouvette principale et un insert de filtration destiné à être inséré dans la structure d'éprouvette principale.

16. Dispositif de dosage de l'activité enzymatique selon l'une quelconque des revendications 3 à 13, dans lequel la structure de support solide se trouve sous la forme d'un dispositif à écoulement latéral supportant ledit substrat biopolymère, et dans lequel le dispositif à écoulement latéral comprend un support rigide portant un tampon d'échantillon et une structure de membrane agencée pour former une trajectoire, dans lequel ledit substrat polymère est agencé sur ledit tampon d'échantillon, ledit tampon d'échantillon n'absorbe sensiblement pas le liquide, de préférence le tampon d'échantillon a une surface hydrophobe supportant le substrat biopolymère.

17. Dispositif de dosage de l'activité enzymatique selon l'une quelconque des revendications 3 à 13, dans lequel ladite structure solide se trouve sous la forme d'un dispositif microfluidique supportant ledit substrat biopolymère, dans lequel ledit dispositif microfluidique comprend au moins un canal d'écoulement comprenant une entrée pour injecter un fluide dans le canal d'écoulement, ledit canal d'écoulement comprenant un site d'échantillonnage comprenant ledit substrat biopolymère.

18. Procédé de détermination de l'activité enzymatique d'un échantillon, le procédé comprenant
• la fourniture d'un dispositif de dosage de l'activité enzymatique comprenant un substrat biopolymère et une structure de support solide supportant ledit substrat biopolymère dans lequel ledit substrat biopolymère comprend un xérogel coloré et insoluble dans l'eau comprenant un réseau de biopolymères réticulés ;
• l'application d'une quantité présélectionnée de l'échantillon sur le substrat biopolymère ;
• le fait de laisser à l'échantillon et au substrat biopolymère un temps d'incubation d'au moins environ 5 secondes, de préférence d'au moins environ 10 secondes, par exemple d'au moins environ 30 secondes, par exemple d'au moins environ 1 minute ;
• l'observation de la libération ou non de colorant et/ou de fragments biopolymères portant le colorant du substrat biopolymère ; et
• la détermination de l'activité enzymatique.
